Europäisches Patentamt

European Patent Office

Office européen des brevets

(11) Veröffentlichungsnummer: **0 520 082 A1**

## (12) EUROPÄISCHE PATENTANMELDUNG

(21) Anmeldenummer: **91110801.7**

(22) Anmeldetag: **28.06.91**

(51) Int. Cl.5: **A61M 16/00**

(43) Veröffentlichungstag der Anmeldung:
**30.12.92 Patentblatt 92/53**

(84) Benannte Vertragsstaaten:
**CH DE ES FR GB IT LI NL SE**

(71) Anmelder: **Siemens Elema AB**
**Röntgenvägen 2**
**S-171 95 Solna 1(SE)**

(84) **SE**

(71) Anmelder: **SIEMENS AKTIENGESELLSCHAFT**
**Wittelsbacherplatz 2**
**W-8000 München 2(DE)**

(84) **CH DE ES FR GB IT LI NL**

(72) Erfinder: **Lindén, Dan, Dipl.-Ing.**
**Dianavägen 35**
**S-115 43 Stockholm(SE)**
Erfinder: **Olsson, Sven-Gunnar**
**Villa Fortuna**
**S-232 00 Arlöv(SE)**

(54) **Beatmungsgerät mit vom Exspirationsgasfluss gesteuertem Inspirationsgasfluss.**

(57) Die Erfindung betrifft ein Beatmungsgerät mit vom Exspirationsgasfluß gesteuertem Inspirationsgasfluß. Um Triggerschwierigkeiten insbesondere bei der Beatmung von Neugeborenen zu vermeiden, ist das Beatmungsgerät so gesteuert, daß ein kontinuierlicher Gasfluß durch die Inspirations- und Exspirationsleitung strömt. Der Gasfluß wird dabei in der Expirationsleitung über einen Gasflußsensor gemessen. Erfindungsgemäß wird das Meßsignal zur Steuerung eines Ventils in der Inspirationsleitung derart herangezogen, daß ein konstanter Gasfluß in der Exspirationsleitung aufrecht erhalten wird.

FIG 1

EP 0 520 082 A1

Die Erfindung betrifft ein Beatmungsgerät gemäß dem Oberbegriff des Anspruches 1.

Ein derartiges Beatmungsgerät ist beispielsweise aus dem Ausbildungsheft für den Servoventilator 900 C, Bestellnr. 6979074 E 315 E, von 1983 bekannt. Bei diesem Ventilator sind verschiedene Beatmungsformen einstellbar. Eine dieser Beatmungsformen ist die Spontanatmung mit kontinuierlichem positivem Atemwegdruck (CPAP). CPAP wird insbesondere bei Neugeborenen angewendet. Die Einleitung der Inspirationsphase wird dabei vom Patienten getriggert, wenn beispielsweise bei einem Einatmungsversuch des Patienten der Druck in der Exspirationsleitung ein einstellbares Niveau unterschreitet. Das Inspirationsventil öffnet dann und die Atmung erfolgt ohne weitere Mithilfe des Ventilators, daß heißt der Patient steuert u.a. Atemfrequenz und Tidalvolumen selbst. Ebenso wird in Abhängigkeit von gewissen Parametern die Inspiration beendet und die Exspiration eingeleitet. Das Exspirationsventil reguliert während der ganzen Zeit den exspiratorischen Druck auf einen eingestellten PEEP-Wert (positiver endexspiratorischer Druck), so daß ein kontinuierlicher, positiver Atemwegdruck aufrecht erhalten wird.

Anstelle der Drucktriggerung ist es ebenfalls bereits bekannt, den Ventilator bei definierten Gasflussänderungen zu triggern.

Bei der Behandlung von Kleinkindern und insbesondere Frühgeburten ist diese Triggerung unter Umständen schwierig. Bei druckgesteuerter Triggerung muß das Kind erst einen gewissen Unterdruck erzeugen, bevor der Ventilator eine neue Inspirationsphase startet. Diese notwendige Anstrengung kann die Behandlung derartiger Patienten erschweren. Hinzu kommt, daß es beim Umschalten dieser bekannten Beatmungsgeräte von der Exspiration zur Inspiration zu störenden Verzögerungen kommen kann, da die in den Gasleitungen zwischen Patient und Beatmungsgerät vorhandene Gassäule eine gewisse Trägheit hat. Beim Umschalten von der Exspirationsphase zur Inspirationsphase vergeht daher eine unerwünschte Zeit, bis frisches Atemgas von der Inspirationsleitung zu den Atemwegen und Lungen des Patienten gelangt.

Der vorliegenden Erfindung liegt die Aufgabe zugrunde, ein Beatmungsgerät der eingangs genannten Art so zu verbessern, daß die möglicherweise mit der Triggerung verbundenen Schwierigkeiten wegfallen. Diese Aufgabe wird erfindungsgemäß durch die Merkmale des Anspruchs 1 gelöst.

Im Gegensatz zu den bisherigen Beatmungsgeräten ist das erfindungsgemäße Beatmungsgerät so gesteuert, daß ein kontinuierlicher Gasfluß durch Inspirations- und Exspirationsleitungen strömt. Der Gasfluß wird dabei in der Exspirationsleitung über einen Gasflußsensor gemessen. Dieser Wert wird zur Steuerung des Inspirationsventiles derart herangezogen, daß praktisch ein konstanter Gasfluß durch die Exspirationsleitung aufrecht erhalten wird. Dadurch wird praktisch nicht mehr zwischen Inspiration und Exspiration in Abhängigkeit von Triggerniveaus umgeschaltet. Während der Einatmung durch den Patienten würde normalerweise der Fluß durch die Exspirationsleitung sinken. Das wird unmittelbar durch den Gasflußsensor in dieser Leitung registriert. In Abhängigkeit von diesem Signal wird das Ventil in der Inspirationsleitung derart geöffnet, daß der Fluß durch die Inspirationsleitung erhöht wird und der Gasfluß durch die Exspirationsleitung praktisch auf einem konstanten Niveau gehalten wird. Während der Ausatmung des Patienten reagiert das Gerät umgekehrt.

In Weiterbildung der Erfindung ist vorgesehen, daß das einstellbare Niveau für den Gasfluß in der Exspirationsleitung einem Bypass - Gasfluß entspricht, der aufrecht erhalten wird während der Zeit, wenn der Patient weder ein- noch ausatmet. In einer vorteilhaften Weiterbildung der Erfindung ist vorgesehen, daß beim Überschreiten des Gasflusses in der Exspirationsleitung über das eingestellte Niveau die Öffnung des Inspirationsventils unabhängig von der Differenz zwischen tatsächlichen Gasfluß in der Exspirationsleitung und dem eingestellten Niveau nach einer vorgebbaren Zeitfunktion reduziert wird. Damit ist es beispielsweise während der Exspiration möglich, den Gasfluß in der Inspirationsleitung nach einer bekannten Funktion abnehmen zu lassen oder sogar konstant zu halten. Durch Messen der Gasflüsse sowohl in der Inspirations- als auch in der Exspirationsleitung läßt sich damit einfacher das exspiratorische Gesamtvoumen ermitteln, da praktisch keine Schwankungen des Gasflusses in der Insprirationsleitung auftreten.

Eine einfache Steuerung des Inspirationsgasflusses in Abhängigkeit vom Exspirationsgasfluß läßt sich erfindungsgemäß dadurch erzielen, daß ein Regler mit negativen Feedsback vorgesehen ist, dem als Istwert ein dem eingestellten Niveau entsprechendes Signal und als Sollwert das Signal des Flußsensors zugeführt sind. Das Ausgangssignal dieses Reglers kann direkt zur Steuerung des Inspirationsventiles verwendet werden. Vorteilhafterweise kann jedoch die Steuerung des Inspirationsventiles über einen weiteren Regler erfolgen, dem dann das Ausgangssignal des ersten Reglers als Referenzsignal oder Sollwert zugeführt wird. In Weiterbildung der Erfindung ist vorgesehen, daß als Regler ein I- Regler vorgsehen ist. Dadurch wird erreicht, daß kurzzeitige Flußschwankungen auf der Exspirationsseite die Regelung kaum beeinflussen, wodurch ein Überschwingen des Reglers besser verhindert werden kann.

Weitere Vorteile der Erfindung ergeben sich aus den Unteransprüchen sowie in Zusammenhang mit den Ausführungsbeispielen, die im folgenden an Hand von 4 figuren naher beschrieben und erläutert werden. Dabei zeigen

Fig 1. den prinzipiellen mechanischen Aufbau eines Ventilators,

Fig 2. ein Blockschaltbild der erfindungsgemäßen Regelung des Inspirationsgasflusses,

Fig 3. eine mögliche Ausführungsform einer Schaltung entsprechend dem Blockschaltbild gemäß Fig 2 und die Figuren 4A, 4B und 5 den zeitlichen Verlauf der Gasflüsse durch die Exspirations- und Inspirationsleitung.

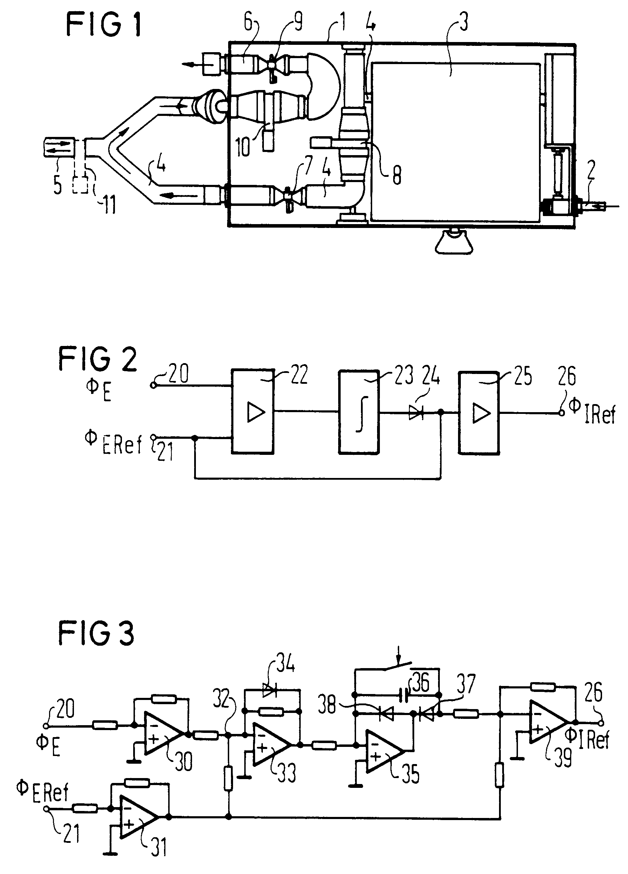

In der Fig 1 ist ein Beatmungsgerät 1 mit seinen wesentlichen mechanischen/pneumatischen Teilen dargestellt. Das Atemgas gelangt über eine Leitung 2 in einen Vorratsbehälter 3, beispielsweise einen Balg. Von diesem wird es über eine Inspirationsleitung 4 einer mit einem nicht dargestellten Patienten verbundenen Leitung 5 zugeführt. Diese Leitung 5 ist andererseits mit der Exspirationsleitung 6 verbunden. Die Pfeile in den Leitungen geben die Richtung des Gasflusses an. Die Inspirationsleitung 4 ist mit einem Ventil 7 versehen und mit einem Gasflußsensor 8. Die Exspirationsleitung 6 ist ebenso mit einem Ventil 9 und einem Gasflußsensor 10 versehen. Darüber hinaus kann an jeder Leitung auch noch ein hier nicht dargestellter Drucksensor angeschlossen sein. Die Signale der Fluß- bzw. Drucksensoren sind auf eine hier nicht dargestellte Steuervorrichtung geschaltet, die in Abhängigkeit von diesen Signalen und in Abhängigkeit von der eingestellten Beatmungsform und den vorgegebenen Randbedingungen die Ventile 7 bzw. 9 steuert. Gestrichelt ist weiterhin ein Gasflußsensor 11 in der Leitung 5 dargestellt, dessen Signal ebenfalls auf die Steuervorrichtung geschaltet ist.

In Fig. 2 ist in einem einfachen Blockschaltbild das Prinzip des erfindungsgemäßen Beatmungsgerätes dargestellt. An einem Anschluß 20 liegt dabei ein dem Gasfluß $\Phi_E$ in der Exspirationsleitung entsprechendes Signal an. An dem Anschluß 21 liegt ein dem eingestellten Niveau des gewünschten Gasflusses $\Phi_{ERef}$ in der Exspirationsleitung entsprechendes Referenzsignal an. In einem Differenzverstärker 22 wird die Differenz beider Signale, daß heißt die Abweichung des tatsächlichen Flusses vom eingestellten Fluß, gebildet und unter Umständen auch noch verstärkt. Dieses Differenzsignal wird auf einen nachfolgenden Integrator 23 geschaltet, dessen Ausgangssignal über eine Diode 24 auf einen weiteren Verstärker 25 geschaltet ist, auf den zusätzlich noch einmal das Referenzsignal gegeben ist. Am Ausgang 26 dieses Verstärkers 25 liegt dann ein Signal vor, das zur Steuerung des Ventils in der Inspirationsleitung verwendet wird und hier mit $\Phi_{IRef}$ bezeichnet ist.

Die Wirkungsweise dieser Schaltung ist folgende: geht man zunächst davon aus, daß der tatsächliche Fluß $\Phi_E$ auf dem eingestellten Niveau liegt, so wird die Differenz und damit das Ausgangssignal des Verstärkers 22 Null. Auch der Integrator 23 liefert kein Ausgangssignal. Das einzige am Verstärker 25 anliegende Eingangssignal ist somit das Referenzsignal, das nach einer Verstärkung zur Steuerung des Ventils 7 in der Inspirationsleitung dient und den Fluß durch dieses Ventil konstant hält. Tritt nun beispielsweise durch den Einatmungsversuch eines Patienten der Fall auf, daß $\Phi_E$ gegenüber $\Phi_{ERef}$ sinkt, wird die Differenz verstärkt und integriert und, solang der Integrationswert positiv ist, über die Diode 24 auf den Verstärker 25 gegeben und dort zu dem Referenzsignal addiert. Das Ausgangssignal $\Phi_{IRef}$ wird damit in Abhängigkeit vom tatsächlichen Gasfluß durch die Exspirationsleitung relativt zum festgelegten Niveau geändert. Die Änderung soll dabei dazu führen, daß der Gasfluß auf der Inspirationsseite erhöht wird, um die Gasentnahme durch den Patienten zu kompensieren und den Gasfluß auf der Exspirationsseite wieder auf das vorgegebene Niveau anzuheben.

Im Ramen der Erfindung ist es selbstverständlich auch möglich, die Diode 24 gemäß Fig. 2 wegzulassen und damit den Gasfluß auf der Inspirationsseite in beiden Richtungen dem Gasfluß auf der Exspirationsseite nachzuführen. Das würde dann dazu führen, daß bei sinkendem Gasfluß in der Exspirationsleitung der Gasfluß durch die Inspirationsleitung erhöht wird und umgekehrt. Durch die Diode 24 wird die Nachregelung des Inspirationsflusses Praktisch auf die Inspirationsphase beschränkt, daß heißt auf die Phase, bei der durch Einatmungsbestrebungen des Patienten der Gasfluß in der Exspirationsleitung unter das eingestellte Niveau sinken will. Während der Exspirationsphase erhält man dann einen definierten, keinen plötzlichen Schwankungen unterworfenen Wert für den Inspirationsgasfluß, wodurch die Ermittlung des exspiratorischen Gesamtvolumens erleichtert wird.

In der folgenden Fig. 3 ist ein Schaltungsbeispiel für die Erzeugung eines Regelsignals für das Ventil 7 in der Inspirationsleitung entsprechend der Fig. 2 dargestellt. Das dem tatsächlichen Fluß $\Phi_E$ entsprechende Signal wird über den Anschluß 20 auf einen ersten Verstärker 30 gegeben. Entsprechend wird das Referenzsignal über den Anschluß 21 auf einen Verstärker 31 gegeben. Beide dienen zur Signalaufbereitung. Im Punkt 32 sind die Ausgänge dieser beiden Verstärker zusammengeführt, so daß dort ein Signal vorliegt, daß der Differenz der Ausgangssignale beider Verstärker entspricht. Polarität und Verstärkungsfaktoren sind dabei so gewählt, daß für den Fall, daß der tatsächliche Fluß dem eingestellten Fluß entspricht, die Differenz Null wird. Das Signal im Punkt 32 ist auf einen Verstärker 33 geschaltet, der im wesentlichen dem Verstärker 22 gemäß Fig. 2 entspricht. Im Rückkopplungszweig dieses Verstärkers ist dem Widerstand eine Diode 34 parallel geschaltet. Diese Diode bewirkt, daß bei negativer Eingangsspannung am Verstärker 33 die Diode sperrt und die Verstärkung entsprechend den eingestellten Widerständen erfolgt. Am Ausgang liegt dann ein gegenüber dem Eingang invertiertes, verstärktes Signal vor. Wird das Eingangssignal hingegen positiv, so wird die Diode 34 leitend und am Ausgang des Verstärkers 33 liegt unabhängig von der Größe des Eingangssignales ein konstantes Ausgangssignal von beispielsweise -0,6 Volt vor.

Das Ausgangssignal des Verstärkers 33 ist auf einen Integrator 35 geschaltet, der im Prinzip dem Integrator 23 gemäß Fig. 2 entspricht. Im Rückkopplungszweig dieses Integrators ist ein Kondensator 36 mit einer Diode 37 in Reihe geschaltet. Eine weitere Diode 38 ist dazu parallel geschaltet. Solange das Eingangssignal am Intergrator 35 positiv ist, sperrt die Diode 38 und der Kondensator 36 wird aufgeladen. Liegt am Eingang die negative Spannung vor-0,6 Volt, so wird der Kondensator mit dieser konstanten Spannung entladen, aber nicht in umgekehrter Richtung aufgeladen, da die Diode 38 dann leitet.

Die Ausgangsspannung des Integrators 35 wird auf einen weiteren Verstärker 39 geschaltet, auf den gleichzeitig das vom Verstärker 31 kommende Referenzsignal geschaltet ist. Am Eingang dieses Verstärkers 39 liegt somit die Summe des über den Verstärker 31 verstärkten Referenzsignales und des Ausgangssignales des Integrators 35 vor, die nach der Verstärkung durch den Verstärker 39 am Anschluß 26 das Referenzsignal $\Phi_{IRef}$ für die Öffnung des Ventils in der Inspirationsleitung darstellt.

Wie bereits erwähnt, kann auf die Dioden 34,37 und 38 verzichtet werden und die Änderung des Gasflußes in der Exspirationsleitung in beiden Richtungen, sowohl in positiver als auch negativer, durch Änderung des Inspirationsgasflusses entsprechend kompensiert werden. Darüber hinaus ist es im Rahmen der Erfindung auch möglich, neben der Integralreglung noch weitere Reglungen wie Proportionalreglung hinzuzuschalten oder die Integralreglung durch eine Proportionalreglung zu ersetzen.

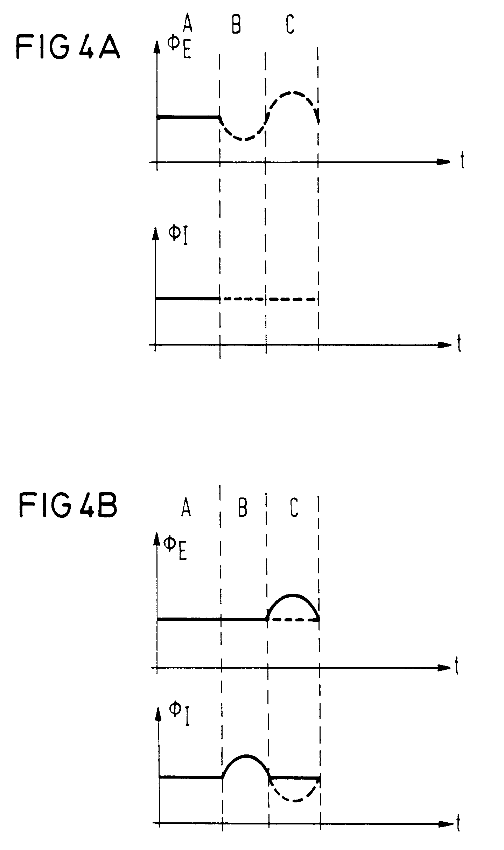

In den Figuren 4A und 4B ist der Gasfluß in der Exspirations - bzw. Inspirationsleitung über der Zeit aufgetragen. Die Zeitachse ist dabei in drei Abschnitte A,B,C unterteilt.

In der Figur 4A wird im Abschnitt A angenommen, daß der Patientengasfluß Null ist, daß heißt das der Patient weder ein- noch ausatmet. In diesem fall ist der Exspirationsgasfluß $\Phi_E$ gleich dem Inspirationsgasfluß $\Phi_I$ und konstant. Im Abschnitt B ist der Patientengasfluß negativ, daß heißt der Patient atmet ein. Wenn man davon ausgeht, daß der Inspirationsgasfluß unverändert konstant bleibt, wie im unteren Teil der Fig. 4A gestrichelt angedeutet, so sinkt der Exspirationsgasfluß entsprechend dem negativen Patientengasfluß. Das ist ebenfalls gestrichelt angedeutet. Im Abschnitt C wird angenommen, daß der Patientengasfluß positiv ist, daß heißt, daß der Patient ausatmet. Wiederum unter der Voraussetzung, daß der Inspirationsgasfluß konstant gehalten wird, steigt nunmehr der Exspirationsgasfluß entsprechend.

In der Fig. 4B sind die Gasflüsse für das erfindungsgemäße Beatmungsgerät dargestellt. Für den Abschnitt A ändert sich gegenüber der Fig. 4A nichts. Im Abschnitt B hingegen bleibt der Exspirationsgasfluß nunmehr konstant, da bei der ersten Abweichung von diesem konstanten Niveau der Inspirationsgasfluß zur Kompensation entsprechend erhöht wird. Im Abschnitt C mit positiven Patientengasfluß steigt gemäß dem bevorzugten Ausführungsbeispiel der Exspirationsgasfluß an und der Inspirationsgasfluß wird konstant gehalten. Gestrichelt ist in diesem Bereich jedoch auch die Möglichkeit dargestellt, daß die Regelung auch für diesen Fall durchgreift und den Exspirationsgasfluß konstant hält.

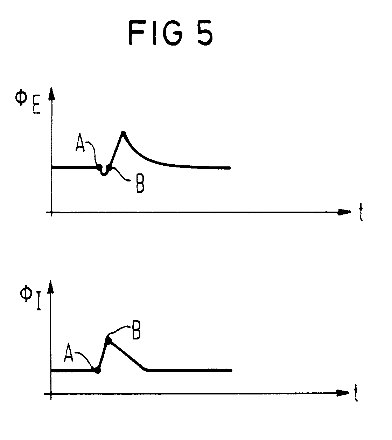

Die Figuren 4A und 4B zeigen den zeitlichen Verlauf der Gasflüsse lediglich schematisch. In der folgenden Figur 5 ist beispielhaft dargestellt, wie der Verlauf des Exspirations- und des Inspirationsgasflusses in der Praxis verlaufen kann.

Zunächst sei wieder ein konstanter Gasfluß gegeben, d.h. der Patient atmet weder ein noch aus. Im Punkt A beginnt er einzuatmen. Das führt zunächst zu einem kurzen Absinken des Exspirationsgasflusses und einem dadurch hervorgerufenen ansteigenden Inspirationsgasfluß entsprechend der erfindungsgemäßen Regelung. Im Punkt B erreicht der Exspirationsgasfluß wieder das vorgegebene konstante Niveau. Ab diesem Zeitpunkt sinkt der nunmehr zu große Inspirationsgasfluß nach einer vorgegebenen Zeitfunktion, im vorliegenden Beispiel linear, während der Exspirationsgas fluß zunächst noch ansteigt, dann aber auch auf das vorgegebene Nieveau zurückkehrt.

Das Atemvolumen des Patienten läßt sich durch Integration der Differenz beider Gasflüsse bestimmen.

Im Rahmen der Erfindung ist es ebenso möglich, das Vetil in der Inspirationsleitung nicht über den Gasfluß durch die Exspirationsleitung, sondern durch den Patientengasfluß zu steuern. Ist der Patientengasfluß negativ, daß heißt atmet der Patient ein, wird das Inspirationsventil entsprechend geöffnet. Ist der Patientengasfluß positiv, bleibt die Öffnung des Inspirationsventils entweder konstant oder sie ändert sich entsprechend einer vorgebbaren Zeitfunktion oder sie ändert sich in Abhängigkeit vom Patientengasfluß. Der Patientengasfluß kann mit einem Gasflußsensor 11 in der gemeinsamen Leitung 5 gemäß Fig. 1 bestimmt werden.

Bezugszeichenliste

| | |
|---|---|
| 1. | Beatmungsgerät |
| 2. | Leitung |
| 3. | Vorratsbehälter |
| 4. | Inspirationsleitung |
| 5. | Leitung |
| 6. | Exspirationsleitung |
| 7. | Ventil |
| 8. | Gasflußsensor |
| 9. | Ventil |
| 10. | Gasflußsensor |
| 11. | Gasflußsensor |
| 20. | Anschluß |
| 21. | Anschluß |
| 22. | Differenzverstärker |
| 23. | Integrator |
| 24. | Diode |
| 25. | Verstärker |
| 26. | Ausgang |
| 30. | Verstärker |
| 31. | Verstärker |
| 32. | Punkt |
| 33. | Verstärker |
| 34. | Diode |
| 35. | Integrator |
| 36. | Kondensator |
| 37. | Diode |
| 38. | Diode |
| 39. | Verstärker |

**Patentansprüche**

1. Beatmungsgerät mit einer Inspirations- und einer Exspirationsleitung (4,6) zum Anschließen an die Atemwege von Menschen oder Tieren, einem in der Inspirationsleitung (4) vorgesehenen ersten Ventil (7), mit dessen Hilfe der Gasfluß durch die Inspirationsleitung (7) einstellbar ist, mindestens einem Gasflußsensor (10:11) in der Exspirationsleitung (6) oder einer von Exspirations- und Inspirationsleitung zum Patienten führenden gemeinsamen Leitung, (5) sowie einer Steuervorrichtung zur Betätigung des ersten Ventils (7), wobei auf diese Steuervorrichtung zumindest das Ausgangssignal des Gasflußsensors (10;11) geschaltet ist, **dadurch gekennzeichnet,** daß das Ventil (7) in der Inspirationsleitung (4) in Abhängigkeit vom Signal des Gasflußsensors (10;11) derat gesteuert wird, daß zumindest bei sinkendem Gasfluß in der Exspirationsleitung (6) unter ein vorgebbares Niveau der Gasfluß durch die Inspirationsleitung (4) derart erhöht wird, daß der Gasfluß durch die Exspirationsleitung (6) auf das Niveau zurückgebracht wird.

2. Beatmungsgerät nach Anspruch 1, **dadurch gekennzeichnet,** daß das vorgebbare Niveau einem Bypass Gasfluß entspricht.

3. Beatmungsgerät nach Anspruch 1 oder 2, **dadurch gekennzeichnet,** daß beim Überschreiten des Gasflusses in der Exspirationsleitung (6) über das Niveau die Öffnung des Ventils (7) unabhängig von der Differenz zwischen tatsächlichem Gasfluß in der Exspirationsleitung (6) und dem Niveau nach einer vorgebbaren Zeitfunktion reduziert wird.

4. Beatmungsgerät nach einem der Ansprüche 1 bis 3, **dadurch gekennzeichnet,** daß die Steuervorrichtung einen Regler mit negativem Feedback enthält, auf den sowohl ein dem Niveau entsprechendes Referenzsignal als auch das Ausgangssignal des Gasflußsensors (10;11) geschaltet sind und der in Abhängigkeit von der Differenz zwischen beiden Signalen ein Signal zur Steuerung des Inspirationsventiles (7) erzeugt.

5. Beatmungsgerät nach Anspruch 4, **dadurch gekennzeichnet,** daß also Regler ein I - Regler vorgesehen ist.

6. Beatmungsgerät nach Anspruch 4 oder 5, **dadurch gekennzeichnet,** daß sich bei einer Polarität der Differenz das Ausgangssignal des I - Reglers nach einer vorgebbaren Zeitfunktion unabhängig von der Größe der Differenz ändert.

7. Beatmungsgerät nach einem der Ansprüche 1 bis 6, **gekennzeichnet durch** einen ersten Verstärker (22;23), auf den das Referenzsignal und das Ausgangssignal des Gasflußsensors derart geschaltet sind, daß am Eingang dieses Verstärkers die Differenz zwischen beiden Signalen anliegt, einem Integrator (23;35), auf dessen Eingang das Ausgangssignal des ersten Verstärkers geschaltet ist, sowie einen weiteren Verstärker (25;39), auf den das Ausgangssignal des Integrators und das Referenzsignal derart geschaltet sind, das am Eingang dieses weiteren Verstärkers die Summe beider Signale anliegt, und wobei das Ausgangssignal des weiteren Verstärkers zur Steuerung des Ventils (7) in der Inspirationsleitung (4) dient.

8. Beatmungsgerät nach Anspruch 7, wobei das erste Ventil (7) über einen Regelkreis mit negativem Feedback derart gesteuert wird, daß die Differenz zwischen dem Signal eines weiteren Gasflußsensors (8) in der Inspirationsleitung (4) und einem weiteren Referenzsignal, das einem gewünschten Gasfluß durch die Inspirationsleitung (4) entspricht, gegen Null strebt, **dadurch gekennzeichnet,** daß als weiteres Referenzsignal das Ausgangssignal des weiteren Verstärkers (25;39) dient.

9. Beatmungsgerät nach Anspruch 7 oder 8, **dadurch gekennzeichnet,** daß zwischen Integrator und weiteren Verstärker eine Diode geschaltet ist.

10. Beatmungsgerät nach einem der Ansprüche 7 bis 9, **dadurch gekennzeichnet,** daß als erster Verstärker (33) ein Operationsverstärker mit einem Widerstand im Rückkopplungszweig vorgesehen ist, wobei dem Widerstand eine Diode (34) parallel geschaltet ist.

FIG 1

FIG 2

$\Phi_E$ 20

$\Phi_{ERef}$ 21

22 23 24 25 26

$\Phi_{IRef}$

FIG 3

$\Phi_E$ 20

$\Phi_{ERef}$ 21

30 31 32 33 34 35 36 37 38 39 26

$\Phi_{IRef}$

7

FIG 4A

FIG 4B

# FIG 5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 402 951 (M AKASHI)<br>* Spalte 4, Zeile 25 - Zeile 40 *<br>* Spalte 5, Zeile 48 - Spalte 6, Zeile 22 *<br>* Abbildung 3 *<br>--- | 1,2 | A61M16/00 |
| E | EP-A-0 459 647 (PURITAN-BENNETT CORP.)<br>* Spalte 3, Zeile 32 - Zeile 42 *<br>* Spalte 5, Zeile 28 - Zeile 43 *<br>* Spalte 6, Zeile 4 - Zeile 33 *<br>* Abbildung 1 *<br><br>----- | 1,2 | |
| | | | **RECHERCHIERTE SACHGEBIETE (Int. Cl.5)**<br><br>A61M |

Der vorliegende Recherchenbericht wurde für alle Patentansprüche erstellt

| Recherchenort | Abschlußdatum der Recherche | Prüfer |
|---|---|---|
| DEN HAAG | 27 FEBRUAR 1992 | VEREECKE A. |

EPO FORM 1503 03.82 (P0403)